# EUROPÄISCHE PATENTSCHRIFT

(11) **EP 1 233 736 B1**
(45) Veröffentlichungstag und Bekanntmachung des Hinweises auf die Patenterteilung: **31.03.2004**
(21) Anmeldenummer: 00975663.6
(22) Anmeldetag: 13.11.2000
(51) Int. Cl.: A61H 39/00, A61N 1/32

(54) **PUNKTUAL-STIMULATIONS-THERAPIEGERÄT**
THERAPY APPLIANCE FOR PUNCTUAL STIMULATION
APPAREIL DE THERAPIE PAR STIMULATION PONCTUELLE

(30) Priorität: 12.11.1999 AT 78699 U
(43) Veröffentlichungstag der Anmeldung: 28.08.2002
(73) Patentinhaber: SZELES, Josef Constantin, A-1190 Wien (AT)
(72) Erfinder: SZELES, Josef Constantin, A-1190 Wien (AT)
(74) Vertreter: Weinzinger, Arnulf, Dipl.-Ing.
(86) Internationale Anmeldenummer: PCT/AT2000/000297
(87) Internationale Veröffentlichungsnummer: WO 2001/035897

(56) Entgegenhaltungen:
- DE-U- 29 720 785
- US-A- 5 012 816
- US-A- 5 957 951
- PATENT ABSTRACTS OF JAPAN vol. 017, no. 142 (C-1038), 23. März 1993 (1993-03-23) & JP 04 314459 A (MATSUSHITA ELECTRIC WORKS LTD), 5. November 1992 (1992-11-05)

## Beschreibung

Die Erfindung bezieht sich auf ein Punktual-Stimulations-Therapiegerät mit einem tragbaren batteriebetriebenen Behandlungsstromgenerator, welcher mindestens eine über eine flexible Leitung angeschlossene Elektrode speist, die bei unter der Hautoberfläche liegenden Rezeptorenbereichen anzuordnen ist, wobei dieser Behandlungsstromgenerator in einem kleinen, am Körper zu tragenden Gehäuse angeordnet ist.

Die US-A-5957951 beschreibt ein Gerät zur elektrischen Akupunktur-Therapie, welches Gerät ein kleines, am Körper zu tragendes Gehäuse aufweist, an dessen an den Körper des zu behandelnden Patienten anzulegender Seite mehrere Gruppen von Elektroden fest positioniert angeordnet sind. Zur Lieferung des Therapiestromes sind im Gerät Batterien angeordnet und es können die einzelnen Gruppen der Elektroden wahlweise an die Batterien angeschaltet werden. Zur intermittierenden Anschaltung der Batteriespannung an die einzelnen Gruppen der Elektroden ist auch ein Mikroprozessor in Betracht gezogen.

Die JP-A-4314459 beschreibt ein Therapiegerät, welches zur Behandlung von Patienten niederfrequente Energie abgeben soll. Dieses Gerät ist, damit es auf gekrümmte Oberflächenbereiche eines zu behandelnden Patienten aufgelegt werden kann, mit einem in Art eines aufgeschlagenen Buches ausgebildeten Gehäuse versehen. An diesem Gehäuse ist auch eine Antenne zum Empfang hochfrequenter Steuersignale angeordnet, welche mittels einer Fernbedienungseinheit erzeugt werden.

Die Elektro-Punktual-Stimulations-Therapie, zu der auch verschiedene Formen der Elektroakupunktur zu zählen sind, kann bei verschiedenen Gesundheitsstörungen, wie z.B. Allergien, Asthma, Adipositas, Schmerzzuständen, mit Erfolg angewendet werden. Bei einer Elektro-Punktual-Stimulations-Therapie wird häufig eine sich über mehrere Stunden oder auch mehrere Tage erstreckende Behandlung als günstig erachtet. Dabei soll auch die Mobilität der zu behandelnden Patienten möglichst wenig eingeschränkt werden und die Behandlung nach Möglichkeit im Rahmen eines weitgehend normalen Lebensablaufes, einschließlich einer Teilnahme an Erwerbsarbeit, möglich sein. So ist es eine Zielsetzung, die der vorliegenden Erfindung zugrundeliegt, ein möglichst einfach aufgebautes und leicht herstellbares Therapiegerät zu schaffen, welches auf einfache Weise am Körper, und zwar insbesondere im Ohrbereich, positioniert werden kann und dort, ohne störend in Erscheinung zu treten, ohne zusätzliche Maßnahmen guten Halt findet und längere Zeit getragen werden kann, und es soll auch während der Behandlung vom Gerät her möglichst kein nachteiliger mechanischer Einfluss auf die Elektrodenzuleitungen und auf den Sitz der Elektroden ausgeübt werden. Das zu schaffende Gerät soll unter Berücksichtigung der vorgenannten Faktoren bzw. zu erzielenden Eigenschaften die Möglichkeit bieten, innerhalb einer Vielfalt von Varianten einen für den jeweiligen Anwendungsfall günstigen Verlauf des Behandlungsstromes zu wählen bzw. einzustellen, wobei es möglich sein soll, eine Auswahl zu treffen unter verschiedenen Kurvenformen, Impulsformen und Frequenzen des Behandlungsstromes, unter verschiedenen zeitabhängigen Variationen der Intensität des Behandlungsstromes und auch unter verschiedenen Möglichkeiten der Zeitdauer von Stromflussintervallen und Stromflusspausen im Zuge von Behandlungssequenzen und auch hinsichtlich der Dauer von Ruhepausen zwischen Behandlungssequenzen.

Das erfindungsgemäße Punktual-Stimulations-Therapiegerät eingangs erwähnter Art ist dadurch gekennzeichnet, dass der Behandlungsstromgenerator mit einem den Behandlungsstromverlauf mindestens hinsichtlich Wellenform und Amplitude des Behandlungsstromes steuernden programmierbaren Mikrochip versehen ist. Durch diese Ausbildung kann der vorstehend angeführten Zielsetzung auf einfache Weise mit geringem Aufwand gut entsprochen werden. Es wird durch die Ausbildung des Behandlungsstromgenerators mit einem programmierbaren Mikrochip die Möglichkeit geschaffen, auf einfache Weise einen jedem einzelnen Behandlungsfall optimal entsprechenden Verlauf des Behandlungsstromes zu wählen, ohne dass man hierfür eine größere Anzahl von weiteren Schaltungselementen einsetzen muss, so dass der Behandlungsstromgenerator mit sehr geringen Abmessungen ausgeführt werden kann, was eine entsprechend kleine und gewichtsmäßig leichte Ausführung des Gerätes ermöglicht, das dadurch problemlos am Körper, insbesondere im Ohrbereich, platziert werden kann, in einer Weise, welche einen guten stabilen Sitz des Gerätes über längere Zeiträume sichert und auch die Gefahr unerwünschter mechanischer Einflüsse auf die zu den Punktual-Stimulations-Elektroden führenden Verbindungsleitungen, und damit weiter auch auf diese Elektroden selbst, gering zu halten vermag.

Es besteht die Möglichkeit, mit einem sehr kleinen und verhältnismäßig kostengünstig zu realisierenden Aufbau einen Behandlungsstromgenerator zu erstellen, der auch bei lang dauernden Behandlungen unter Anwendung verschiedener Behandlungsstromverläufe keiner wiederholten Bedienung von Seiten des zu behandelnden Patienten bedarf, so dass am Gerät keine dessen Sitz allfällig beeinträchtigenden Einstellhandlungen vorgenommen werden müssen, und man kann durch die Steuerung des Behandlungsstromverlaufes mittels des programmierbaren Mikrochips durch einfaches Programmieren desselben eine Vielzahl von Varianten des Behandlungsstromverlaufes herstellen. Man kann dabei nicht nur hinsichtlich Wellenform und Amplitude vielfältige Verlaufsformen des Behandlungsstromes herstellen, sondern auch eine Vielzahl von Behandlungsstrommustern hinsichtlich des Vorsehens kurzzeitiger Stromunterbrechungen in einzelnen Behandlungssequenzen und hinsichtlich längerer Pausen zwischen Behandlungssequenzen bilden. Eine die diesbezüglichen vielfältigen Möglichkeiten ausschöpfende Ausführungsform ist dadurch gekennzeichnet, dass der programmierbare Mikrochip zur digitalen Synthese der jeweils gewünschten Wellenform und Amplitude des Behandlungsstromes jeweils von außen programmierbar ist. Eine andere Ausführungsform, welche dadurch gekennzeichnet ist, dass der programmierbare Mikrochip mit einem Speicher versehen ist, in dem mehrere Wellenformen und Amplituden des Behandlungsstromes gespeichert und wahlweise abrufbar sind, bietet die Möglichkeit, dass ein Therapeut auf einfache Weise aus einer Gruppe von Behandlungsstromverläufen jeweils einen für den spezifischen Fall besonders geeignet erscheinenden Behandlungsstromverlauf auswählt und gegebenenfalls im Zuge einer längeren Behandlungsserie durch Änderung der Auswahl den Behandlungsverlauf beeinflusst. Korrespondierend dazu kann man auch vorsehen, dass der programmierbare Mikrochip mit einem Speicher versehen ist, in dem je mehrere Werte für die Zeitdauer von in Behandlungssequenzen anzuwendenden Stromflussintervallen und Stromflusspausenintervallen und/oder für die Zeitdauer von zwischen Behandlungssequenzen vorzusehenden Ruhepausen gespeichert und wahlweise abrufbar sind. In diesem Fall ist wieder eine einfache Auswahl aus mehreren Möglichkeiten ausführbar. Ein größeres Spektrum an Möglichkeiten hinsichtlich der Realisierung bestimmter Pausen im Zuge von Behandlungssequenzen und längerer Ruhezeiten zwischen aufeinanderfolgen den Behandlungssequenzen gibt eine Ausführungsform, welche dadurch gekennzeichnet ist, dass der programmierbare Mikrochip zur Steuerung der Länge von Stromflussintervallen und Stromflusspausenintervallen im Zuge von Behandlungssequenzen und/oder der Dauer von zwischen Behandlungssequenzen vorgesehenen Ruhepausen programmierbar ist.

Gemäß einer bevorzugten Ausbildung des erfindungsgemäßen Punktual-Stimulations-Therapiegerätes ist der programmierbare Mikrochip ein Mikroprozessor bzw. Mikrocontroller. Es ergeben sich dadurch Vorteile hinsichtlich der Durchführung von Programmierungen und hinsichtlich des Erzielens einer breiten Vielfalt von Varianten des Behandlungsstromverlaufes; auch ist diese Ausbildung einfach mit relativ geringem Aufwand realisierbar.

Für einen über längere Zeiträume stabilen guten Sitz des erfindungsgemäßen Gerätes an einer Stelle der Hautoberfläche und gleichzeitig auch zur Erfüllung der Zielsetzung, dass das Gerät den Träger auch über längere Zeiträume nicht wesentlich stört, ist eine Ausführungsform dieses Gerätes vorteilhaft, welche dadurch gekennzeichnet ist, dass das Gehäuse des Gerätes annähernd die geometrische Form einer von einem Ellypsoid geschnittenen Kalotte aufweist und an der Basisfläche der Kalotte eine seitlich über diese Basisfläche hinausragende, an der der Kalotte abgewandten Seite klebehaftend ausgebildete flexible Flächenelektrodenscheibe trägt, welche als Basiselektrode zur Zufuhr des Behandlungsstromes vorgesehen und mit dem Behandlungsstromgenerator verbunden ist. Es wird durch die Kalottenform nicht nur ein meist gewünschtes unauffälliges Aussehen erreicht, sondern auch der Einfluss von den Sitz des Gerätes belastenden mechanischen Kräften gemildert und weiter die Stabilität des Sitzes des Gerätes durch die seitlich hinausragende klebehaftend ausgebildete Flächenelektrodenscheibe besonders unterstützt. Durch das seitliche Hinausragen dieser Scheibe kann deren Rand auf einfache Weise an die betreffende Körperstelle satt angedrückt werden, und es kann sich die Scheibe problemlos an die Körperstelle anschmiegen, wodurch ein besonders guter Sitz erzielt wird.

Hinsichtlich der Ausbildung der Flächenelektrodenscheibe ist es aus baulichen Gründen und für die angestrebte Schmiegsamkeit vorteilhaft, wenn man vorsieht, dass die Flächenelektrodenscheibe eine aus elektrisch isolierendem flexiblen Material gebildete Scheibe ist, welche mit ihrer einen Flächenseite am Gehäuse des Gerätes sitzt, und an ihrer anderen Flächenseite, die vom Gehäuse des Gerätes abgewandt ist, eine elektrisch leitende Schicht aufweist. Hierbei ist es baulich günstig, wenn die elektrisch leitende Schicht der Flächenelektrodenscheibe mit durch das Isoliermaterial der Scheibe hindurchführenden Kontaktverbindern an den Behandlungsstromgenerator angeschlossen ist. Weiter ist es für einen guten elektrischen und mechanischen Kontakt von Vorteil, wenn zur Intensivierung des elektrischen und mechanischen Kontaktes zwischen der die elektrisch leitende Schicht aufweisenden Seite der Flächenelektrodenscheibe und der Hautoberfläche ein elektrisch leitendes Gel vorgesehen ist.

Für die Positionierung des Gerätes an verschiedenen Körperstellen, insbesondere hinter dem Ohr, ist es günstig, wenn man vorsieht, dass die Flächenelektrodenscheibe an mindestens einer Stelle ihres Umfangsrandes eine Einbuchtung aufweist und sonst im Wesentlichen ringsum gleich breit über den Rand der Basisfläche der Kalotte hinausragt.

Eine besonders baulich günstige Ausführungsform des erfindungsgemäßen Gerätes ist dadurch gekennzeichnet, dass das Gehäuse des Gerätes einen inneren Gehäuseteil, der den mit einem programierbaren Mikrochip versehenen Behandlungsstromgenerator, Speisebatterien, eine elektrische Verbindungen bildende Printplatte und Behandlungsstromausgangskontakte aufnimmt, und einen äußeren Gehäuseteil in Form einer kalottenartigen Kappe, welche den inneren Gehäuseteil überdeckend auf diesen aufschnappbar ist, aufweist, wobei die Flächenelektrodenscheibe am Rand des inneren Gehäuseteiles fixiert ist.

In funktioneller Hinsicht ist es oft günstig, und zwar insbesondere dann, wenn das erfindungsgemäß ausgebildete Gerät nur für eine einmalige Verwendung vorgesehen werden soll, wenn man vorsieht, dass im inneren Gehäuseteil ein in die Stromzufuhr des Behandlungsstromgenerators eingefügter Schalter angeordnet ist, der sich im Ruhezustand in "AUS"-Stellung befindet und durch Andrücken in die "EIN"-Stellung versetzbar ist, wobei ein zum Andrücken vorgesehener Stift am äußeren Gehäuseteil vorgesehen ist und der Schalter mit diesem Stift beim Aufschnappen des äußeren Gehäuseteils auf den inneren Gehäuseteil in die "EIN"-Stellung setzbar ist.

Die Erfindung wird nachstehend anhand von Beispielen unter Bezugnahme auf die schematisch gehaltene Zeichnung weiter erläutert.

In der Zeichnung zeigt
Fig.1 eine Ausführungsform eines erfindungsgemäß ausgebildeten Punktual-Stimulations-Therapiegerätes in Draufsicht,
Fig.2 dieses Gerät in einem Schnitt gemäß der Linie II-II in Fig.1, und
Fig.3 wesentliche Teile dieses Gerätes in einer Explosionsdarstellung.

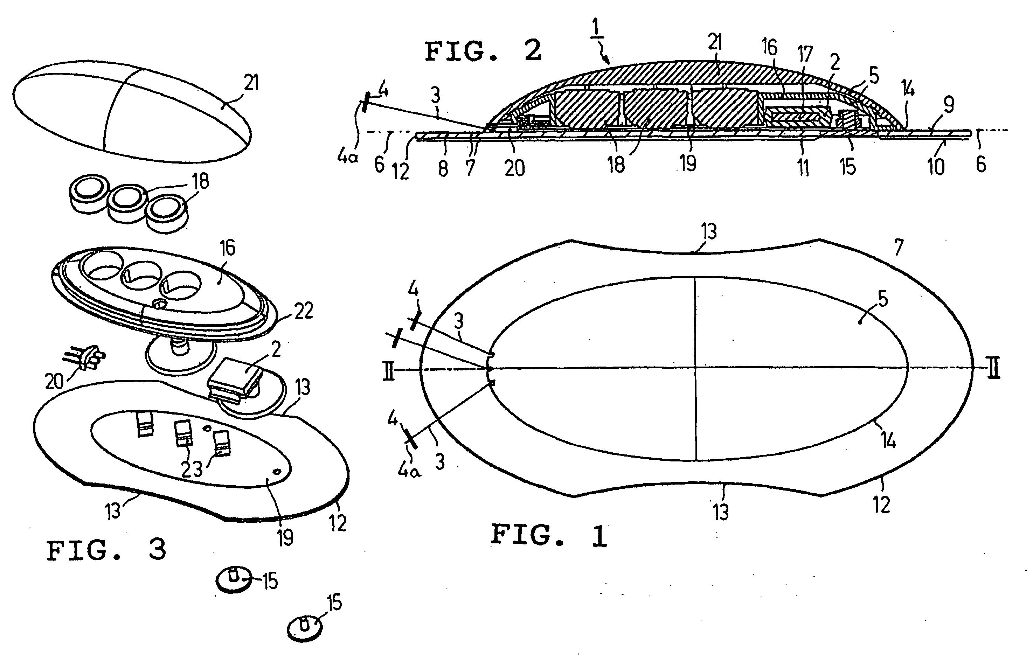

Die in der Zeichnung dargestellte Ausführungsform eines erfindungsgemäß ausgebildeten Punktual-Stimulations-Therapiegerätes 1 kann z.B. eine Größe von einem Viertel der Darstellungen in den Fig.1 und 2 haben. Das Gerät 1 ist mit einem batteriebetriebenen Behandlungsstromgenerator 2 versehen, welcher über flexible Leitungen 3 angeschlossene Elektroden 4 speist. Solche Elektroden 4, welche zur Stimulation von Rezeptorenbereichen vorgesehen sind, welche sich an verschiedenen Stellen unter der Hautoberfläche befinden, sind im dargestellten Fall mit Nadelspitzen 4a versehen, welche an Stellen, die über solchen Rezeptorenbereichen liegen, in die Hautoberfläche eingestochen werden. Es sind aber auch andere als die dargestellten Elektrodenformen möglich. Der Behandlungsstromgenerator 2 ist in einem kleinen, am Körper zu tragenden Gehäuse 5 angeordnet, in dem sich auch die zur Speisung des Behandlungsstromgenerators erforderlichen Batterien befinden. Der Behandlungsstromgenerator 2 ist mit einem den Behandlungstromverlauf steuernden programierbaren Mikrochip 17 versehen.

Das Gehäuse 5 des Punktual-Stimulations-Therapiegerätes 1 hat annähernd die geometrische Form einer von einem Ellipsoid geschnittenen Kalotte und trägt an der Basisfläche 6 der Kalotte eine seitlich über diese Basisfläche hinausragende Flächenelektrodenscheibe 7, welche an der der Kalotte abgewandten Seite klebehaftend ausgebildet ist. Die Flächenelektrodenscheibe 7 ist als Basiselektrode zur Zufuhr des Behandlungsstromes vorgesehen, d.h. dass sie die Behandlungsstromkreise, die vom Behandlungsstromgenerator 2 zu den Elektroden 4 und weiter zu den zu stimulierenden Rezeptorenbereichen führen, wieder zum Behandlungsstromgenerator 2 schließt, mit dem diese Flächenelektrodenscheibe 7 elektrisch verbunden ist.

Vorzugsweise ist die Flächenelektrodenscheibe 7 eine aus elektrisch isolierenden flexiblen Material gebildete Scheibe 8, welche mit ihrer einen Flächenseite 9 am Gehäuse 5 des Gerätes 1 sitzt und an ihrer anderen Flächenseite 10, die vom Gehäuse 5 des Gerätes abgewandt ist, eine elektrisch leitende Schicht 11 aufweist. Diese elektrisch leitende Schicht 11 der Flächenelektrodenscheibe 7 ist im dargestellten Fall mit durch das Isoliermaterial der Scheibe 8 hindurchführenden Kontaktverbindern 15 an den Behandlungsstromgenerator 2 angeschlossen. Zur Intensivierung des elektrischen und mechanischen Kontaktes zwischen der die elektrisch leitende Schicht 11 aufweisenden Seite 10 der Flächenelektrodenscheibe 7 und der Hautoberfläche, auf die das Gerät aufgesetzt wird, um die erwähnten über die Rezeptorenbereiche führenden Stromkreise der Behandlungsströme zu schließen, wird vorzugsweise ein in der Zeichnung nicht näher dargestelltes elektrisch leitendes Gel vorgesehen.

In vielen Fällen erweist es sich bei Anwendung einer Elektro-Stimulations-Therapie von Rezeptorenbereichen als vorteilhaft, wenn man im Bereich der Ohrmuschel liegende Rezeptorenbereiche stimuliert. Es ist dabei sowohl hinsichtlich der Führung der Leitungen 3, durch welche Behandlungsströme zu den Elektroden 4 gelangen, als auch hinsichtlich der über die Rezeptorenbereiche fließenden Stimulationsströme günstig, wenn man das mit einer Flächenelektrode versehene Gerät im Bereich des Ohres an der Hautoberfläche anordnet. Hierfür ist es günstig, wenn man die Flächenelektrodenscheibe 7 an mindestens einer Stelle ihres Umfangsrandes 12 mit einer Einbuchtung 13 versieht, wodurch das Gerät sehr einfach dicht hinter dem Ohr platziert werden kann, was sowohl hinsichtlich der häufig gewünschten Unauffälligkeit als auch aus Gründen eines guten Haltes sowie auch hinsichtlich der Elektrostimulation Vorteile bietet. Im Übrigen ragt die Flächenelektrodenscheibe 7 im Wesentlichen ringsum gleich breit über den Rand 14 der Basisfläche 6 der Kalotte hinaus. Durch dieses Hinausragen der Flächenelektrodenscheibe 7 kann diese flexible Scheibe problemlos satt an die Hautoberfläche der Anbringungsstelle des Gerätes angedrückt werden, wobei sich durch die klebehaftende Ausbildung der Scheibe ein guter und sicherer Halt trotz der in der Regel vorliegenden Unebenheiten der Hautoberfläche ergibt. Es ist dabei günstig, dass die der Kalotte zugewandte Flächenseite 9 der Scheibe elektrisch isolierend ist, so dass auch bei eingeschaltetem Gerät an dieser Scheibe wirkende Andrückmanipulationen keine unmittelbare Auswirkung auf die elektrischen Verhältnisse haben.

Im dargestellten Fall hat das Gehäuse 5 des Gerätes 1 einen inneren Gehäuseteil 16, der den mit einem programmierbaren Mikrochip 17 versehenen Behandlungsstromgenerator 2, weiter Speisebatterien 18, eine elektrische Verbindungen des Gerätes bildende Printplatte 19 und Behandlungsstromausgangskontakte 20 aufnimmt, und weiter einen äußeren Gehäuseteil 21 in Form einer kalottenartigen Kappe, welche den inneren Gehäuseteil 16 überdeckend auf diesen aufschnappbar ist. Die Flächenelektrodenscheibe 7 ist am Rand 22 des inneren Gehäuseteiles 16 fixiert, z.B. angeklebt oder angeschweißt. Kontaktverbinder 15 führen von der äußeren Flächenseite 10 der Flächenelektrodenscheibe 7 durch das Isoliermaterial der Scheibe 8 hindurch zum Behandlungsstromgenerator 2 und schließen damit diese elektrisch leitende Schicht 11 an den Behandlungsstromgenerator 2 an. Verbindungskontakte 23 stellen elektrische Verbindungen zwischen den einzelnen Speisebatterien 18 und von diesen Speisebatterien zum Behandlungsstromgenerator 2 her. Im inneren Gehäuseteil 16 ist beim dargestellten Ausführungsbeispiel weiter ein nicht näher dargestellter Schalter angeordnet, der in die Stromzufuhr des Behandlungsstromgenerators 2 eingefügt ist, und der sich im Ruhezustand in "AUS"-Stellung befindet und durch Andrücken in die "EIN"-Stellung versetzbar ist, wobei ein zum Andrücken vorgesehener, gleichfalls nicht näher dargestellter Stift am äußeren Gehäuseteil 22 vorgesehen ist und der Schalter mit diesem Stift beim Aufschnappen des äußeren Gehäuseteiles 22 auf den inneren Gehäuseteil 16 in die "EIN"-Stellung setzbar ist. Vorteilhaft sieht man auch am oder im inneren Gehäuseteil 16 oder an der Printplatte 19 einen Schnittstellenanschluss vor, über den der programmierbare Mikrochip 17, welcher wie oben erwähnt vorteilhaft in Form eines Mikrocontrollers bzw. Mikroprozessors ausgebildet ist, dem jeweiligen Stimulationsziel entsprechend programmiert werden kann.

## Patentansprüche

1. Punktual-Stimulations-Therapiegerät (1) mit einem tragbaren batteriebetriebenen Behandlungsstromgenerator (2), welcher mindestens eine über eine flexible Leitung (3) angeschlossene Elektrode (4) speist, die bei unter der Hautoberfläche liegenden Rezeptorenbereichen anzuordnen ist, wobei dieser Behandlungsstromgenerator (2) in einem kleinen, am Körper zu tragenden Gehäuse (5) angeordnet ist, **dadurch gekennzeichnet, dass** der Behandlungsstromgenerator (2) mit einem den Behandlungsstromverlauf mindestens hinsichtlich Wellenform und Amplitude des Behandlungsstromes steuernden programmierbaren Mikrochip (17) versehen ist.

2. Punktual-Stimulations-Therapiegerät nach Anspruch 1, **dadurch gekennzeichnet, dass** der programmierbare Mikrochip (17) zur digitalen Synthese der jeweils gewünschten Wellenform und Amplitude des Behandlungsstromes jeweils von außen programmierbar ist.

3. Punktual-Stimulations-Therapiegerät nach Anspruch 1, **dadurch gekennzeichnet, dass** der programmierbare Mikrochip (17) mit einem Speicher versehen ist, in dem mehrere Wellenformen und Amplituden des Behandlungsstromes gespeichert und wahlweise abrufbar sind.

4. Punktual-Stimulations-Therapiegerät nach einem der Ansprüche 1 bis 3, **dadurch gekennzeichnet, dass** der programmierbare Mikrochip (17) zur Steuerung der Länge von Stromflussintervallen und Stromflusspausenintervallen im Zuge von Behandlungssequenzen und/oder der Dauer von zwischen Behandlungssequenzen vorgesehenen Ruhepausen programmierbar ist.

5. Punktual-Stimulations-Therapiegerät nach einem der Ansprüche 1 bis 3, **dadurch gekennzeichnet, dass** der programmierbare Mikrochip (17) mit einem Speicher versehen ist, in dem je mehrere Werte für die Zeitdauer von in Behandlungssequenzen anzuwendenden Stromflussintervallen und Stromflusspausenintervallen und/oder für die Zeitdauer von zwischen Behandlungssequenzen vorzusehenden Ruhepausen gespeichert und wahlweise abrufbar sind.

6. Punktual-Stimulations-Therapiegerät nach einem der Ansprüche 1 bis 5, **dadurch gekennzeichnet, dass** der programmierbare Mikrochip (17) ein Mikroprozessor bzw. Mikrocontroller ist.

7. Punktual-Stimulations-Therapiegerät nach einem der vorhergehenden Ansprüche, **dadurch gekennzeichnet, dass** das Gehäuse (5) des Gerätes (1) annähernd die geometrische Form einer von einem Ellypsoid geschnittenen Kalotte aufweist und an der Basisfläche (6) der Kalotte eine seitlich über diese Basisfläche hinausragende, an der der Kalotte abgewandten Seite klebehaftend ausgebildete flexible Flächenelektrodenscheibe (7) trägt, welche als Basiselektrode zur Zufuhr des Behandlungsstromes vorgesehen und mit dem Behandlungsstromgenerator (2) verbunden ist.

8. Punktual-Stimulations-Therapiegerät nach Anspruch 7, **dadurch gekennzeichnet, dass** die Flächenelektrodenscheibe (7) eine aus elektrisch isolierendem flexiblen Material gebildete Scheibe (8) ist, welche mit ihrer einen Flächenseite (9) am Gehäuse (5) des Gerätes (1) sitzt, und an ihrer anderen Flächenseite (10), die vom Gehäuse (5) des Gerätes abgewandt ist, eine elektrisch leitende Schicht (11) aufweist.

9. Punktual-Stimulations-Therapiegerät nach Anspruch 8, **dadurch gekennzeichnet, dass** die elektrisch leitende Schicht (11) der Flächenelektrodenscheibe (7) mit durch das Isoliermaterial der Scheibe (8) hindurchführenden Kontaktverbindern (15) an den Behandlungsstromgenerator (2) angeschlossen ist.

10. Punktual-Stimulations-Therapiegerät nach einem der Ansprüche 7 bis 9, **dadurch gekennzeichnet, dass** zur Intensivierung des elektrischen und mechanischen Kontaktes zwischen der die elektrisch leitende Schicht (11) aufweisenden Seite (10) der Flächenelektrodenscheibe (7) und der Hautoberfläche ein elektrisch leitendes Gel vorgesehen ist.

11. Punktual-Stimulations-Therapiegerät nach einem der Ansprüche 7 bis 10, **dadurch gekennzeichnet, dass** die Flächenelektrodenscheibe (7) an mindestens einer Stelle ihres Umfangsrandes (12) eine Einbuchtung (13) aufweist und sonst im Wesentlichen ringsum gleich breit über den Rand (14) der Basisfläche (6) der Kalotte hinausragt.

12. Punktual-Stimulations-Therapiegerät nach einem der Ansprüche 7 bis 11, **dadurch gekennzeichnet, dass** das Gehäuse (5) des Gerätes (1) einen inneren Gehäuseteil (16), der den mit einem programierbaren Mikrochip (17) versehenen Behandlungsstromgenerator (2), Speisebatterien (18), eine elektrische Verbindungen bildende Printplatte (19) und Behandlungsstromausgangskontakte (20) aufnimmt, und einen äußeren Gehäuseteil (21) in Form einer kalottenartigen Kappe, welche den inneren Gehäuseteil (16) überdeckend auf diesen aufschnappbar ist, aufweist, wobei die Flächenelektrodenscheibe (7) am Rand (22) des inneren Gehäuseteiles (16) fixiert ist.

13. Punktual-Stimulations-Therapiegerät nach Anspruch 12, **dadurch gekennzeichnet, dass** im inneren Gehäuseteil (16) ein in die Stromzufuhr des Behandlungsstromgenerators (2) eingefügter Schalter angeordnet ist, der sich im Ruhezustand in "AUS"-Stellung befindet und durch Andrücken in die "EIN"-Stellung versetzbar ist, wobei ein zum Andrücken vorgesehener Stift am äußeren Gehäuseteil (21) vorgesehen ist und der Schalter mit diesem Stift beim Aufschnappen des äußeren Gehäuseteils (21) auf den inneren Gehäuseteil (16) in die "EIN"-Stellung setzbar ist.

## Claims

1. A punctual stimulation therapy appliance (1) comprising a portable, battery-operated treatment current generator (2) feeding at least one electrode (4) connected via a flexible line (3), which electrode is to be arranged at receptor regions located under the surface of the skin, this treatment current generator (2) being located in a small housing (5) to be worn on the body, **characterized in that** the treatment current generator (2) is provided with a programmable microchip (17) which controls the treatment current course at least with regard to the waveform and amplitude of the treatment current.

2. A punctual stimulation therapy appliance according to claim 1, **characterized in that** the programmable microchip (17) may be programmed from the outside in each case for the digital synthesis of the respective desired waveform and amplitude of the treatment current.

3. A punctual stimulation therapy appliance according to claim 1, **characterized in that** the programmable microchip (17) is provided with a memory in which several waveforms and amplitudes of the treatment current are stored and optionally can be called.

4. A punctual stimulation therapy appliance according to any one of claims 1 to 3, **characterized in that** the programmable microchip (17) may be programmed for controlling the length of the current flow intervals and current flow pause intervals in the course of treatment sequences and/or the duration of rest pauses provided between the treatment sequences.

5. A punctual stimulation therapy appliance according to any one of claims 1 to 3, **characterized in that** the programmable microchip (17) is provided with a memory in which in each case several values for the duration of current flow intervals and current flow pause intervals to be used in treatment sequences and/or for the duration of rest pauses to be provided between treatment sequences can be stored and optionally called.

6. A punctual stimulation therapy appliance according to any one of claims 1 to 5, **characterized in that** the programmable microchip (17) is a microprocessor, or microcontroller, respectively.

7. A punctual stimulation therapy appliance according to any one of the preceding claims, **characterized in that** the housing (5) of appliance (1) approximately has the geometrical form of a calotte cut from an ellipsoid, and, at the base plane (6) of the calotte carries a flexible surface electrode disk (7) laterally projecting beyond the base plane and designed to be adhesive at its side facing away from the calotte, which surface electrode disk is provided as a base electrode for supplying the treatment current and connected to the treatment current generator (2).

8. A punctual stimulation therapy appliance according to claim 7, **characterized in that** the surface electrode disk (7) is a disk (8) formed of electrically insulating flexible material which with its one surface side (9) is seated on the housing (5) of the appliance (1) and at its other surface side (10) which faces away from the housing (5) of the appliance has an electrically conductive layer (11).

9. A punctual stimulation therapy appliance according to claim 8, **characterized in that** the electrically conductive layer (11) of the surface electrode disk (7) is connected to the treatment current generator (2) by contact connectors (15) passing through the insulating material of the disk (8).

10. A punctual stimulation therapy appliance according to any one of claims 7 to 9, **characterized in that** an electrically conductive gel is provided for intensifying the electrical and mechanical contact between the side (10) of the surface electrode disk (7) provided with the electrically conductive layer (11) and the surface of the skin.

11. A punctual stimulation therapy appliance according to any one of claims 7 to 10, **characterized in that** the surface electrode disk (7) on at least one site of its peripheral rim (12) has an indentation (13) and otherwise projects substantially all around with an equal width beyond the rim (14) of the base plane (6) of the calotte.

12. A punctual stimulation therapy appliance according to any one of claims 7 to 11, **characterized in that** the housing (5) of the appliance (1) has an inner housing part (16) accommodating the treatment current generator (2) provided with the programmable microchip (17), feeding batteries (18), a printed board (19) forming electrical connections, and treatment current output contacts (20), and an outer housing part (21) having the shape of a calotte-type cap capable of being snapped on the inner housing part (16) so as to cover the latter, the surface electrode disk (7) being fixed at the rim (22) of the inner housing part (16).

13. A punctual stimulation therapy appliance according to claim 12, **characterized in that** a switch is arranged in the inner housing part (16), which switch is included in the current supply of the treatment current generator (2) and which, in the at-rest position, is in its "OFF" position and can be moved into its "ON" position by pressing thereon, a pin for pressing thereon being provided on outer housing part (21), the switch being movable into its "ON" position by means of this pin when the outer housing part (21) is snapped onto the inner housing part (16).

## Revendications

1. Appareil de thérapie (1) par stimulation ponctuelle comportant un générateur de courant de traitement (2) portable, fonctionnant sur batterie, qui alimente au moins une électrode (4) reliée à un conducteur flexible (3), qui doit être placée dans des zones de récepteurs se trouvant sous la surface supérieure de la peau, ce générateur de courant de traitement (2) étant disposé dans un petit boîtier (5) à porter sur le corps, **caractérisé en ce que** le générateur de courant de traitement (2) est muni d'une micro-puce programmable (17) commandant l'allure du courant de traitement au moins en ce qui concerne la forme d'onde et l'amplitude du courant.

2. Appareil de thérapie par stimulation ponctuelle selon la revendication 1, **caractérisé en ce que** la micro-puce programmable (17 est programmable de l'extérieur à la fois pour la synthèse numérique de la forme d'onde et de l'amplitude souhaitées respectives du courant de traitement.

3. Appareil de thérapie par stimulation ponctuelle selon la revendication 1, **caractérisé en ce que** la micro-puce programmable (17) est pourvue d'une mémoire dans laquelle plusieurs formes d'onde et amplitudes du courant de traitement sont stockées, et peuvent être appelées sélectivement.

4. Appareil de thérapie par stimulation ponctuelle selon l'une des revendications 1 à 3, **caractérisé en ce que** la micro-puce programmable (17) est programmable pour la commande de la longueur des intervalles du flux de courant, et d'intervalles de pause du flux de courant dans les trains de séquences de traitement, et/ou de la durée des pauses de repos prévues entre deux séquences de traitement.

5. Appareil de thérapie par stimulation ponctuelle selon l'une des revendications 1 à 3, **caractérisé en ce que** la micro-puce programmable (17) est munie d'une mémoire dans laquelle sont stockées et peuvent être appelées au choix sélectivement plusieurs valeurs de la durée des intervalles du flux de courant et des intervalles des pauses du flux de courant, et/ou des durées des pauses de repos prévues entre des séquences de traitement.

6. Appareil de thérapie par stimulation ponctuelle selon l'une des revendications 1 à 5, **caractérisé en ce que** la micro-puce programmable (17) est un microprocesseur ou un microcontrôleur.

7. Appareil de thérapie par stimulation ponctuelle selon l'une quelconque des revendications précédentes, **caractérisé en ce que** le boîtier (5) de l'appareil (1) présente la forme géométrique voisine d'une calotte coupée par un ellipsoïde, et porte sur la surface de base (6) de la calotte une rondelle - électrode superficielle (7) agencée de façon flexible, adhésive du côté opposé à la calotte, dépassant latéralement au-dessus de cette surface de base, laquelle rondelle est prévue comme électrode de base pour l'alimentation en courant de traitement et est reliée au générateur (2) de courant de traitement.

8. Appareil de thérapie par stimulation ponctuelle selon la revendication 7, **caractérisé en ce que** la rondelle- électrode superficielle (7) est une rondelle (8) constituée en un matériau flexible électriquement isolant, qui s'appuie sur le boîtier (5) de l'appareil (1) par une surface latérale (9), et qui présente une couche (11) électriquement conductrice sur son autre surface latérale (10), qui est à l'opposé du boîtier (5) de l'appareil.

9. Appareil de thérapie par stimulation ponctuelle selon la revendication 8, **caractérisé en ce que** la couche électriquement conductrice (11) de la rondelle - électrode superficielle (7) est reliée au générateur de courant de traitement (2) par des raccords de contact (15) conduisant au travers du matériau isolant de la rondelle (8).

10. Appareil de thérapie par stimulation ponctuelle selon l'une des revendications 7 à 9, **caractérisé en ce que**, pour l'intensification du contact électrique et mécanique entre le côté (10) de la rondelle - électrode superficielle (7) présentant la couche électriquement conductrice (11) et la surface supérieure de la peau, il est prévu un gel électriquement conducteur.

11. Appareil de thérapie par stimulation ponctuelle selon l'une des revendications 7 à 10, **caractérisé en ce que** la rondelle- électrode superficielle (7) présente une échancrure (13) sur au moins un emplacement de son bord périphérique (12), et par ailleurs dépasse sensiblement d'une largeur identique tout autour du bord (14) de la surface de base (6) de la calotte.

12. Appareil de thérapie à stimulation ponctuelle selon l'une des revendications 7 à 11, **caractérisé en ce que** le boîtier (5) de l'appareil (1) comporte une partie de boîtier intérieur (16) qui reçoit le générateur de courant de traitement (2) pourvu d'une micro-puce programmable (17), des batteries d'alimentation (18), un circuit imprimé (19) formant des liaisons électriques et des contacts de sortie (20) du courant de traitement constituant des liaisons électriques, et une partie de boîtier extérieure (21) sous forme d'un chapeau du type calotte, qui peut être verrouillé par ressort sur la partie de boîtier intérieure (16) en recouvrant celle-ci, et dans lequel la rondelle- électrode superficielle (7) est fixée au bord (22) de la partie de boîtier intérieure (16).

13. Appareil de thérapie à stimulation ponctuelle selon la revendication 12, **caractérisé en ce qu'**un commutateur inséré dans l'alimentation du générateur de courant de traitement (2) est disposé dans la partie de boîtier intérieure (16), et se trouve à l'état de repos dans la position « AUS » et par pression peut être décalé dans la position « EIN », **en ce que** qu'il est prévu sur la partie de boîtier extérieure (21) une goupille pour l'application d'une pression, et le commutateur muni de cette goupille peut être mis sur la position « EIN » lors de l'insertion de la partie de boîtier extérieure (21) sur la partie de boîtier intérieure (16).
